# EUROPÄISCHE PATENTSCHRIFT

(11) **EP 0 820 981 B1**
(45) Veröffentlichungstag und Bekanntmachung des Hinweises auf die Patenterteilung: **18.10.2000**
(21) Anmeldenummer: 97111975.5
(22) Anmeldetag: 14.07.1997
(51) Int. Cl.: C07C 209/26, C07C 217/84, C07C 205/37, C07C 209/36

(54) **Verfahren zur Herstellung von 2-Trifluormethoxy-anilin**
Process for the preparation of 2-trifluoromethoxy-aniline
Procédé pour la préparation de la 2-trifluorométhoxy-aniline

(30) Priorität: 26.07.1996 DE 19630228
(43) Veröffentlichungstag der Anmeldung: 28.01.1998
(73) Patentinhaber: BAYER AG, 51368 Leverkusen (DE)
(72) Erfinder: Lantzsch, Reinhard, Dr., 42115 Wuppertal (DE); Marhold, Albrecht, Dr., 51373 Leverkusen (DE)

(56) Entgegenhaltungen:
- EP-A- 0 054 464
- EP-A- 0 143 769
- EP-A- 0 546 391
- DE-A- 2 024 249
- DE-A- 2 549 900
- WILLIAM SHEPPARD: "Alpha-Fluorinated ethers." JOURNAL OF ORGANIC CHEMISTRY., Bd. 29, Nr. 1, 13.Januar 1964, EASTON US, Seiten 1-11, XP002042236

## Beschreibung

Die Erfindung betrifft ein neues Verfahren und neue Zwischenprodukte zur Herstellung von 2-Trifluormethoxy-anilin, welches als Ausgangsstoff für Wirkstoffe in Medizin und Landwirtschaft bekannt ist.

Es ist bekannt, daß man 2-Trifluormethoxy-anilin erhält, wenn man 1-Chlor-2-trifluormethoxy-benzol mit Ammoniak in Gegenwart eines Katalysators, wie z.B. Kupfer(I)-chlorid, und gegebenenfalls in Gegenwart von Wasser bei Temperaturen zwischen 200°C und 280°C umsetzt (vgl. EP 546391).

Diese Umsetzung erfordert jedoch technisch aufwendige Bedingungen. Sie wird unter hohem Druck und bei hohen Temperaturen durchgeführt, so daß an die benötigten Autoklaven-Materialien hohe Anforderungen gestellt werden müssen. Ausbeuten und Qualitäten der im einzelnen erhaltenen Produkte sind zudem auch nicht immer ganz zufriedenstellend.

Es wurde nun gefunden, daß man 2-Trifluormethoxy-anilin der Formel (I) 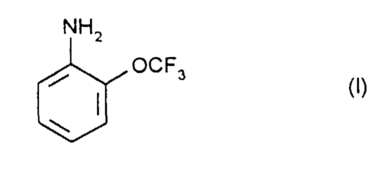 in sehr guten Ausbeuten und in hoher Reinheit erhält, wenn man
in einer ersten Stufe 1,2-Dichlor-4-trifluormethoxy-benzol der Formel (II) 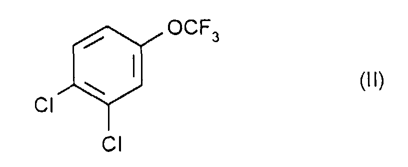 mit einem Nitrierungsmittel, gegebenenfalls in Gegenwart eines Reaktionshilfsmittels und/oder Verdünnungsmittels bei Temperaturen zwischen -20°C und +80°C umsetzt und die hierbei gebildeten Nitrierungsprodukte der allgemeinen Formel (III) 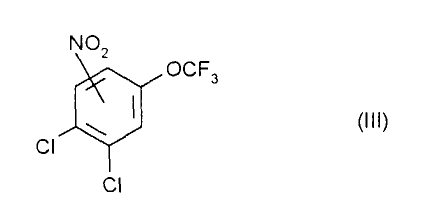 in einer zweiten Stufe mit Wasserstoff in Gegenwart eines Katalysators, in Gegenwart eines Verdünnungsmittels und gegebenenfalls in Gegenwart eines Reaktionshilfsmittels bei Temperaturen zwischen 0°C und 200°C umsetzt,
wobei gegebenenfalls auch intermediär gebildete Dichlor-trifluormethoxy-aniline der allgemeinen Formel (IV) 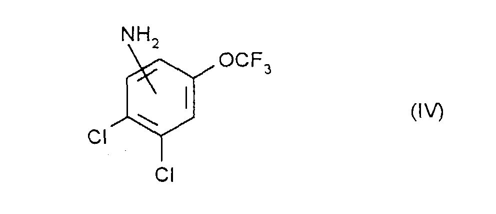 isoliert werden können.

Die allgemeine Formel (III) steht für die Formeln (IIIA) und (IIIB)

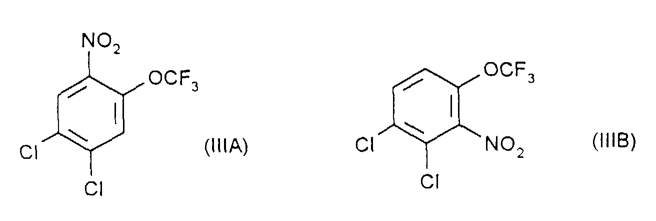

Die allgemeine Formel (IV) steht für die Formeln (IVA) und (IVB)

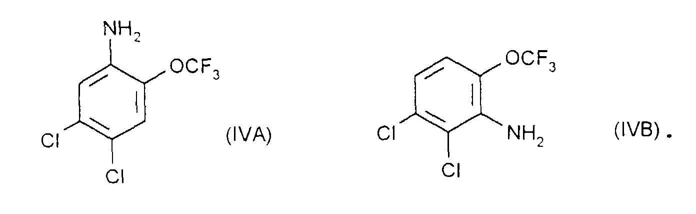

Die Verbindungen der allgemeinen Formel (III) - d.h. die Verbindungen der Formeln (IIIA) und (IIIB) - sowie die Verbindungen der allgemeinen Formel (IV) - d.h. die Verbindungen der Formeln (IVA) und (IVB) - sind noch nicht aus der Literatur bekannt (Aus der DE-A-20 24 249 ist lediglich das Stellungsisomer 2,4-Dichlor-5-methoxyanilin zu den Verbindungen der Formel (IVA) und (IVB) bekannt.); sie sind als neue Stoffe auch Gegenstand der vorliegenden Anmeldung.

Es ist als ausgesprochen überraschend anzusehen, daß die Nitrierung von 1,2-Dichlor-4-trifluormethoxy-benzol der Formel (II) in hoher Selektivität in ortho-Stellung zu der Trifluormethoxy-Gruppierung erfolgt und praktisch kein 1,2-Dichlor-6-nitro-4-trifluormethoxy-benzol gebildet wird, während bei der Nitrierung von 1-Chlor-4-trifluormethoxy-benzol fast gleich große Mengen an 1-Chlor-2-nitro-4-trifluormethoxy-benzol und 1-Chlor-3-nitro-4-trifluormethoxy-benzol entstehen (vgl. J. Org. Chem. 29 (1964), 1-11).

Das erfindungsgemäße Verfahren kann in beiden Stufen auf einfache Weise durchgeführt werden, wobei in der Technik breit genutzte Standardapparaturen verwendet werden können. Es stellt somit eine wertvolle Bereicherung des Standes der Technik dar.

Die beim erfindungsgemäßen Verfahren als Ausgangsstoff zu verwendende Verbindung 1,2-Dichlor-4-trifluormethoxy-benzol der Formel (II) ist bekannt bzw. kann auf bekannte Weise hergestellt werden (vgl. EP 546391; Herstellungsbeispiele).

Die erste Stufe des erfindungsgemäßen Verfahrens wird unter Verwendung eines Nitrierungsmittels durchgeführt. Es kommen hierbei die üblichen Mittel zur Nitrierung aromatischer organischer Verbindungen in Betracht. Hierzu gehört insbesondere Salpetersäure, welche in diversen wässrigen Verdünnungen - z.B. als sog. konz. Salpetersäure (ca. 65%ig) oder als sog. rauchende Salpetersäure (ca. 98%ig) - eingesetzt werden kann.

Die erste Stufe des erfindungsgemäßen Verfahrens wird gegebenenfalls in Gegenwart eines Reaktionshilfsmittels durchgeführt. Als Reaktionshilfsmittel kommen hierbei vorzugsweise Säuren, wie z.B. Schwefelsäure, in Betracht.

Die erste Stufe des erfindungsgemäßen Verfahrens wird gegebenenfalls in Gegenwart eines Verdünnungsmittels durchgeführt. Als solche sind hierbei vor allem relativ inerte organische Lösungsmittel, wie z.B. Methylenchlorid oder Chloroform geeignet. Es können jedoch auch die als Reaktionshilfsmittel bereitserwähnten Säuren gleichzeitig als Verdünnungsmittel verwendet werden.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen -20°C und +80°C, vorzugsweise zwischen -10°C und +60°C, insbesondere zwischen 0°C und +40°C.

Das erfindungsgemäße Verfahren wird in der ersten Stufe im allgemeinen unter Normaldruck durchgeführt. Es ist jedoch auch möglich, die erste Stufe des erfindungsgemäßen Verfahrens unter erhöhtem oder vermindertem Druck - im allgemeinen zwischen 0,1 bar und 10 bar - durchzuführen.

Zur Durchführung der ersten Stufe des erfindungsgemäßen Verfahrens setzt man pro Mol an Ausgangsverbindung der Formel (II) im allgemeinen 1 bis 3 Mol, vorzugsweise 1,1 bis 2,5 Mol an Nitrierungsmittel ein.

In einer bevorzugten Ausführungsform der ersten Stufe des erfindungsgemäßen Verfahrens wird die Ausgangsverbindung der Formel (II) gegebenenfalls zusammen mit einem Reaktionshilfmittel und/oder einem Verdünnungsmittel vorgelegt und das Nitrierungsmittel wird dann langsam eindosiert. Die Reaktionsmischung wird bis zum Ende der Umsetzung bei der erforderlichen Temperatur gerührt und dann auf übliche Weise aufgearbeitet.

Beispielsweise wird nach Ende der Umsetzung mit Eiswasser vermischt, mit einem mit Wasser praktisch nicht mischbaren organischen Lösungsmittel, wie z.B. Methylenchlorid, geschüttelt und die wässrige Phase mehrfach nachextrahiert. Die vereinigten organischen Phasen werden auf übliche Weise getrocknet und filtriert. Vom Filtrat wird das Lösungsmittel unter vermindertem Druck sorgfältig abdestilliert, wobei das Nitrierungsprodukt der Formel (III) - d.h. die Produkte der Formeln (IIIA) und (IIIB) - im Rückstand verbleibt.

Das Zwischenprodukt der Formel (III) kann im allgemeinen ohne weitere Reinigung zur Umsetzung gemäß der zweiten Stufe des erfindungsgemäßen Verfahrens eingesetzt werden; die Komponenten der Formeln (IIIA) und (IIIB) können aber auch nach üblichen Methoden, z.B. durch Destillation unter stark vermindertem Druck, isoliert werden.

Die Hydrierung gemäß der zweiten Stufe des erfindungsgemäßen Verfahrens wird in Gegenwart eines Katalysators durchgeführt. Als Katalysatoren kommen vorzugsweise die bei katalytischen Hydrierungen üblichen Metall-Katalysatoren - gegebenenfalls auf geeigneten Trägermaterialien - in Betracht. Hierzu gehören vorzugsweise (Raney-)Cobalt, (Raney-)Nickel, Palladium und Platin (letztere gegebenenfalls auf einem Trägermaterial wie z.B. Aktivkohle, Tonerde, Kieselgur oder Aluminiumoxid).

Die Hydrierung von anderen chlorierten Nitrobenzolen als den beiden Zwischenprodukten der Formel (IIIA) und (IIIB) ist aus der EP-A-0 054 464 und der EP-A-254 990 bekannt. Die EP-A-0 054 464 beschreibt zudem die vorteilhafte Anwesenheit von Dichlorbenzolderivaten im Hydrierungsgemisch bei der Hydrierung ähnlicher ortho-Anilinderivate wie den Erfindungsgemäßen.

Für den Fall, daß die Intermediate der allgemeinen Formel (IV) isolierbar erhalten werden sollen, wird vorzugsweise (Raney-)Nickel als Katalysator in Gegenwart einer organischen, die Dehalogenierung inhibierenden Verbindung, wie z.B. Thiodiglycol, eingesetzt.

Die zweite Stufe des erfindungsgemäßen Verfahrens wird in Gegenwart eines Verdünnungsmittels durchgeführt. Als Verdünnungsmittel kommen hierbei vorzugsweise Wasser und organische Lösungsmittel in Betracht, insbesondere Alkohole, wie Methanol, Ethanol, n- oder i-Propanol. n-, i-, s- oder t-Butanol; Ether, wie Methyl-t-butylether, Methyl-t-pentylether, Ethylengylcol-dimethylether oder Tetrahydrofuran; Etheralkohole, wie Ethylenglycol-monomethylether oder -monoethylether,ferner Kohlenwasserstoffe, wie Hexan, Cyclohexan, Methylcyclohexan, Toluol oder Xylole, sowie auch Gemische der genannten Lösungsmittel.

Alkohole, insbesondere Methanol und Ethanol, werden als Verdünnungsmittel bei der Durchführung der zweiten Stufe des erfindungsgemäßen Verfahrens ganz besonders bevorzugt eingesetzt.

Die zweite Stufe des erfindungsgemäßen Verfahrens wird gegebenenfalls in Gegenwart eines Säureakzeptors als Reaktionshilfsmittel durchgeführt. Als Säureakzeptoren kommen im allgemeinen die üblichen anorganischen oder organischen Basen in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, n- oder i-propanolat, n-, i-, s- oder t-butanolat; weiterhin auch basische organische Stickstoffverbindungen, wie beispielsweise Trimethylamin, Triethylamin, Tripropylamin, Tributylamin, Trioctylamin, Tridodecylamin, Ethyl-diisopropylamin, N,N-Dimethyl-cyclohexylamin, Dicyclohexylamin, Ethyl-dicyclohexylamin, N,N-Dimethyl-anilin, N,N-Dimethylbenzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), und 1,8-Diazabicyclo[5,4,0]-undec-7-en (DBU).

Die Reaktionstemperaturen können bei der Durchführung der zweiten Stufe des erfindungsgemäßen Verfahrens in einem größeren Bereich variiert werden. Im allgemeinen arbeitet man bei Temperaturen zwischen 0°C und 200°C, vorzugsweise zwischen 10°C und 150°C, insbesondere zwischen 20°C und 100°C.

Die zweite Stufe des erfindungsgemäßen Verfahrens wird im allgemeinen unter Normaldruck oder bei erhöhtem Druck, vorzugsweise zwischen 1 bar und 100 bar, insbesondere zwischen 1 bar und 50 bar, durchgeführt.

In einer bevorzugten Ausführungsform der zweiten Stufe des erfindungsgemäßen Verfahrens wird das durch die Formel (III) definierte Gemisch der Zwischenprodukte der Formeln (IIIA) und (IIIB) in einem geeigneten Verdünnungsmittel vorgelegt, ein Katalysator und gegebenenfalls ein Reaktionshilfsmittel werden dazugegeben und es wird - vorzugsweise bei erhöhtem Druck und bei erhöhter Temperatur - auf übliche Weise hydriert. Wenn die Hydrierung beendet ist, wird gegebenenfalls der restliche Wasserstoff mit Stickstoff verdrängt und die Mischung filtriert. Vom Filtrat wird das Lösungsmittel unter vermindertem Druck abdestilliert. Das im Rückstand verbleibende Rohprodukt kann auf übliche Weise gereinigt und isoliert werden.

Beispielsweise wird mit einem mit Wasser praktisch nicht mischbaren organischen Lösungsmittel, wie z.B. Toluol, und verdünnter wässriger Natronlauge geschüttelt, die organische Phase abgetrennt und durch Destillation unter vermindertem Druck aufgearbeitet.

Die nach dem erfindungsgemäßen Verfahren herzustellende Verbindung 2-Trifluormethoxy-anilin der Formel (I) kann als Zwischenprodukt zur Herstellung von Wirkstoffen in Medizin und Landwirtschaft verwendet werden (vgl. DE 2233845, DE 2601780, DE 2801316, US 4960902, EP 524041, WO 94/14782).

### Herstellungsbeispiele:

### Beispiel 1 (erste Stufe des erfindungsgemäßen Verfahrens)

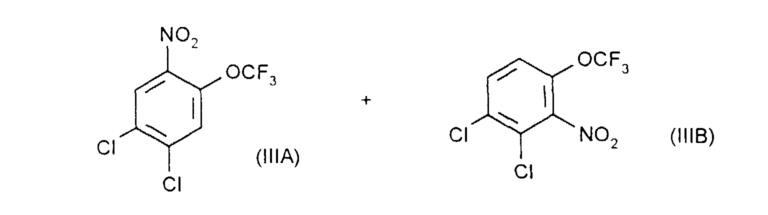

14,1 g (61 mMol) 1,2-Dichlor-4-trifluormethoxy-benzol werden in 183 ml 96%iger Schwefelsäure vorgelegt und 6,1 ml 98%ige Salpetersäure werden tropfenweise dazu gegeben, wobei eine exotherme Reaktion abläuft und die Innentemperatur von anfangs ca. 20°C auf ca. 40°C ansteigt. Die Reaktionsmischung wird ca. 4 Stunden in diesem Temperaturbereich gerührt und dann auf Eiswasser gegossen. Man extrahiert dreimal mit Methylenchlorid, wäscht die vereinigten organischen Phasen mit Wasser, trocknet mit Natriumsulfat und filtriert. Vom Filtrat wird dann das Lösungsmittel im Wasserstrahlvakuum sorgfältig abdestilliert.

Man erhält 15,8 g einer gelben Flüssigkeit, die zu 87,9% aus 1,2-Dichlor-5-nitro-4-trifluormethoxy-benzol (IIIA) und zu 11,4% aus 1,2-Dichlor-3-nitro-4-trifluormethoxy-benzol (IIIB) besteht.

Dies entspricht einer Gesamtausbeute von 93,2% der Theorie.

### Beispiel 2 (zweite Stufe des erfindungsgemäßen Verfahrens)

### (ohne Isolierung der Intermediate der Formel (IV))

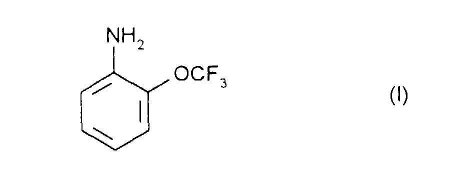

2,5 g (9 mMol) des Produktgemisches aus Beispiel 1 werden in 30 ml Methanol gelöst, mit 0,3 g Palladium auf Kohle (5%ig) versetzt und ca. 5 Stunden lang bei ca. 30°C und 10 bar Wasserstoffdruck hydriert. Der Katalysator wird durch Filtration abgetrennt, mit etwas Methanol nachgewaschen und das Filtrat wird im Wasserstrahlvakuum eingeengt. Der Rückstand wird in Toluol aufgenommen und mit 5%iger Natronlauge verrührt. Die organische Phase wird abgetrennt und durch Destillation unter vermindertem Druck aufgearbeitet.

Man erhält 0,8 g (51% der Theorie) 2-Trifluormethoxy-anilin vom Siedepunkt 60°C (bei 15 mbar, Kugelrohr).

### Beispiel 3 (zweite Stufe des erfindungsgemäßen Verfahrens)

### (ohne Isolierung der Intermediate der Formel (IV))

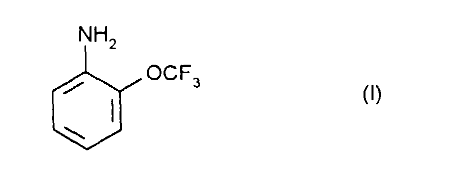

16 g (58 mMol) des Produktgemisches aus Beispiel 1 werden in 300 ml Methanol gelöst, mit 6 g Palladium auf Kohle (5%ig) und mit 22 g (119 mMol) Tributylamin versetzt und ca. 5 Stunden lang bei ca. 30°C und ca. 10 bar Wasserstoffdruck hydriert. Der Katalysator wird durch Filtration abgetrennt, mit etwas Methanol nachgewaschen und das Filtrat wird im Wasserstrahlvakuum eingeengt. Der Rückstand wird mit Wasser versetzt, mit 5%iger Natronlauge alkalisch gestellt und mit Toluol extrahiert. Die organische Phase wird abgetrennt und durch Destillation unter vermindertem Druck aufgearbeitet.

Man erhält 29,7 g eines Destillats, das aus 2-Trifluormethoxy-anilin und Tributylamin besteht, die durch fraktionierte Destillation getrennt werden.
Ausbeute: 8,1 g (78,6% der Theorie).

### Intermediate der Formel (IV):

### Beispiel (IV-1)

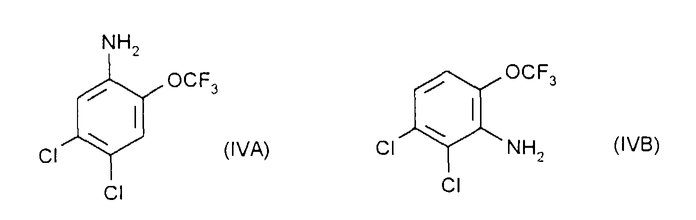

16 g (58 mMol) des Produktgemisches aus Beispiel 1 werden in 400 ml Methanol gelöst und mit einem Tropfen Thiodiglycol [S(CH₂-CH₂OH)₂] versetzt. Nach Zugabe von 2 g Raney-Nickel wird ca. 5 Stunden lang bei ca. 30°C und ca. 5 bar Wasserstoffdruck hydriert. Der Katalysator wird durch Filtration abgetrennt, mit etwas Methanol nachgewaschen und das Filtrat wird im Wasserstrahlvakuum eingeengt. Der Rückstand wird durch Destillation unter vermindertem Druck aufgearbeitet.

Man erhält 11,4 g 4,5-Dichlor-2-trifluormethoxy-anilin (IVA) vom Siedebereich 86°C bis 94°C (bei 15 mbar).

Das isomere 5,6-Dichlor-2-trifluormethoxy-anilin (IVB) kann aus dem Vorlauf der Destillation (bis 85°C, bei 15 mbar) isoliert werden.

### Ausgangsverbindung der Formel (II):

### Beispiel (II-1)

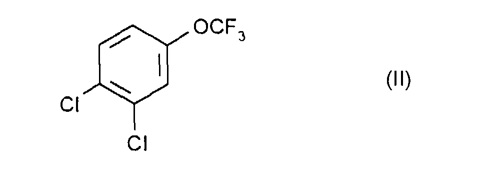

In einem VA-Autoklaven werden 200 g (1,22 Mol) 3,4-Dichlor-phenol und 800 ml Fluorwasserstoff (Hydrogenfluorid) bei 0°C vorgelegt und 800 ml Tetrachlormethan dazu gegeben. Nach Aufdrücken von 15 bar Stickstoff wird unter intensivem Rühren auf 116°C bis 120°C erhitzt und der gebildete Chlorwasserstoff (Hydrogenchlorid) bei ca. 28 bar entspannt. Die Chlorwasserstoff-Entwicklung ist nach ca. 7 Stunden beendet, woraufhin der überschüssige Fluorwasserstoff zusammen mit Trichlorfluormethan und Tetrachlormethan abdestilliert wird. Die anschließende Destillation des Rückstandes bei 60°C bis 86°C (17 mbar) ergibt 213 g eines Produktgemisches aus 19,2 % 1,2-Dichlor-4-trifluormethoxy-benzol und 80,1 % 1,2-Dichlor-4-chlordifluormethoxy-benzol. Dieses Gemisch wird zusammen mit 100 ml Fluorwasserstoff und 1 ml Antimonpentachlorid ca. 3 Stunden auf ca. 125°C erhitzt und der gebildete Chlorwasserstoff bei ca. 25 bar entspannt. Anschließende Destillation liefert 155 g (53 % der Theorie) 1,2-Dichlor-4-trifluormethoxy-benzol vom Siedepunkt 62°C bei 17 mbar.

## Patentansprüche

1. Verfahren zur Herstellung von 2-Trifluormethoxy-anilin der Formel (I), 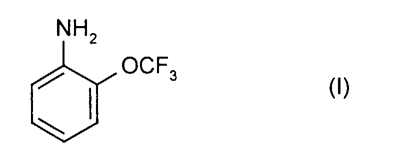 dadurch gekennzeichnet, daß man in einer ersten Stufe 1,2-Dichlor-4-trifluormethoxy-benzol der Formel (II) 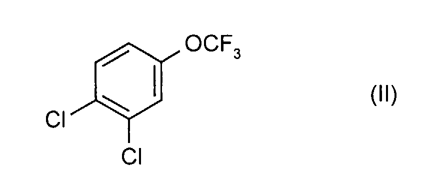 mit einem Nitrierungsmittel bei Temperaturen zwischen -20°C und +80°C umsetzt
und die hierbei gebildeten Nitrierungsprodukte der allgemeinen Formel (IIIA) und (IIIB) 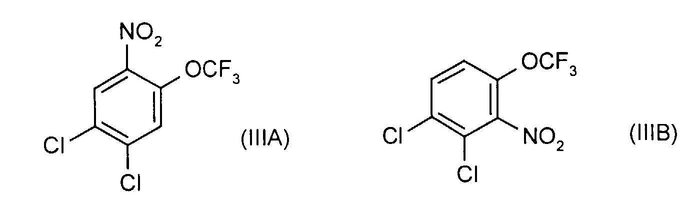 in einer zweiten Stufe mit Wasserstoff in Gegenwart eines Metall-Katalysators, in Gegenwart eines Verdünnungsmittels und bei Temperaturen zwischen 0°C und 200°C umsetzt,
wobei gegebenenfalls auch intermediär gebildete Dichlor-trifluormethoxy-aniline der allgemeinen Formel (IVA) und (IVB) 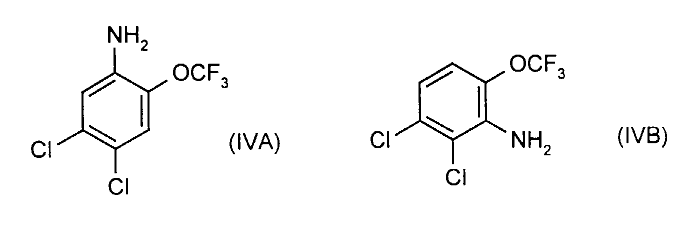 isoliert werden können.

2. Verfahren zur Herstellung von 2-Trifluormethoxy-anilin der Formel (I), 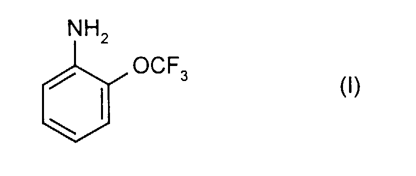 dadurch gekennzeichnet, daß man in einer ersten Stufe 1,2-Dichlor-4-trifluormethoxy-benzol der Formel (II) 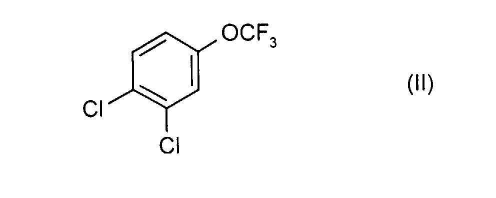 mit einem Nitrierungsmittel in Gegenwart einer Säure und/oder eines inerten organischen Lösungsmittels bei Temperaturen zwischen -20°C und +80°C umsetzt
und die hierbei gebildeten Nitrierungsprodukte der allgemeinen Formel (IIIA) und (IIIB) 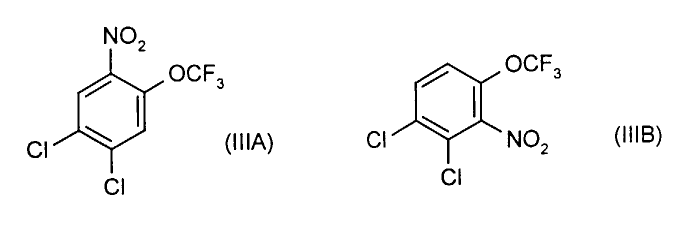 in einer zweiten Stufe mit Wasserstoff in Gegenwart eines Metall-Katalysators, in Gegenwart eines Verdünnungsmittels und gegebenenfalls in Gegenwart eines Säureakzeptors bei Temperaturen zwischen 0°C und 200°C umsetzt,
wobei gegebenenfalls auch intermediär gebildete Dichlor-trifluormethoxy-aniline der allgemeinen Formel (IVA) und (IVB) 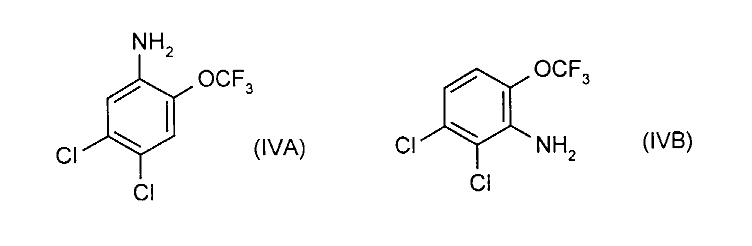 isoliert werden können.

3. Verfahren nach Anspruch 1, dadurch gekennzeichnet, daß die erste Verfahrensstufe bei Temperaturen zwischen -10°C und +60°C durchgeführt wird.

4. Verfahren nach Anspruch 2, dadurch gekennzeichnet, daß die erste Verfahrensstufe bei Temperaturen zwischen 0°C und +40°C durchgeführt wird.

5. Verfahren naach Anspruch 1, dadurch gekennzeichnet, daß die zweite Verfahrensstufe bei Temperaturenm zwischen 10°C und 150°C durchgeführt wird.

6. Verfahren nach Anspruch 4, dadurch gekennzeichnet, daß die zweite Verfahrensstufe bei Temperaturen zwischen 20°C und 100°C durchgeführt wird.

7. Verfahren nach Anspruch 1, dadurch gekennzeichnet, daß die zweite Verfahrensstufe bei einem Druck im Bereich von 1 bar bis 100 bar durchgeführt wird.

8. Verfahren nach Anspruch 6, dadurch gekennzeichnet, daß die zweite Verfahrensstufe bei einem Druck im Bereich von 1 bar bis 50 bar durchgeführt wird.

9. 1,2-Dichlor-5-nitro-4-trifluormethoxy-benzol der Formel (IIIA) und 1,2-Dichlor-3-nitro-4-trifluormethoxy-benzol der Formel (IIIB)

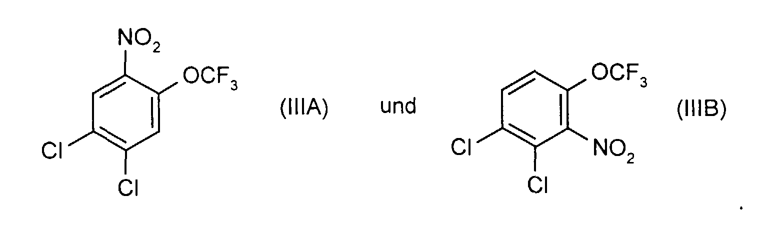

10. 4,5-Dichlor-2-trifluormethoxy-anilin der Formel (IVA) und 5,6-Dichlor-2-trifluormethoxy-anilin der Formel (IVB)

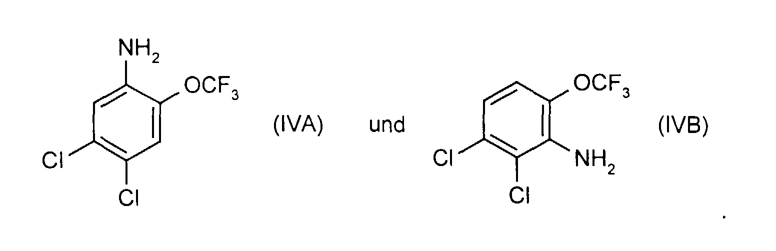

## Claims

1. Process for preparing 2-trifluoromethoxy-aniline of the formula (I) 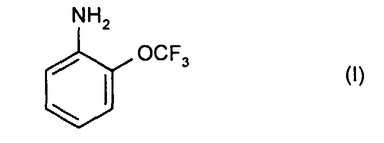 characterized in that in a first step 1,2-dichloro-4-trifluoromethoxy-benzene of the formula (II) 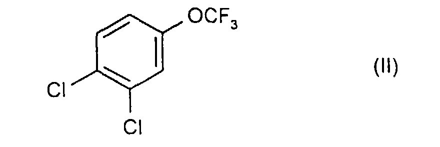 is reacted with a nitrating agent at temperatures between -20°C and +80°C
and the resulting nitration products of the general formulae (IIIA) and (IIIB) 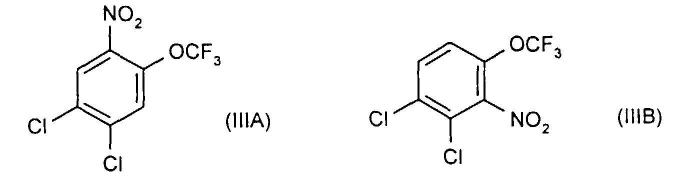 are reacted in a second step with hydrogen in the presence of a metal catalyst, in the presence of a diluent and at temperatures between 0°C and 200°C,
it being possible to isolate the intermediate dichloro-trifluoromethoxy-anilines of the general formulae (IVA) and (IVB) 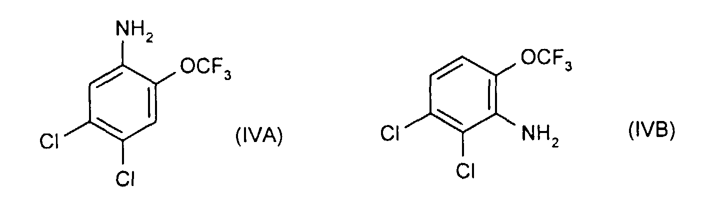 if so desired.

2. Process for preparing 2-trifluoromethoxy-aniline of the formula (I) 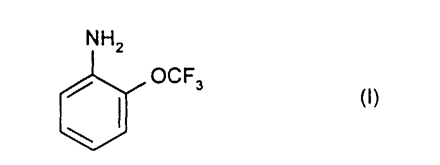 characterized in that in a first step 1,2-dichloro-4-trifluoromethoxy-benzene of the formula (II) 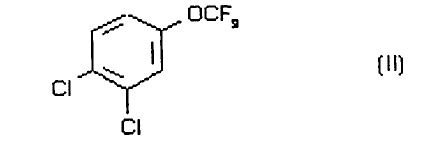 is reacted with a nitrating agent in the presence of an acid and/or of an inert organic solvent at temperatures between -20°C and +80°C
and the resulting nitration products of the general formulae (IIIA) and (IIIB) 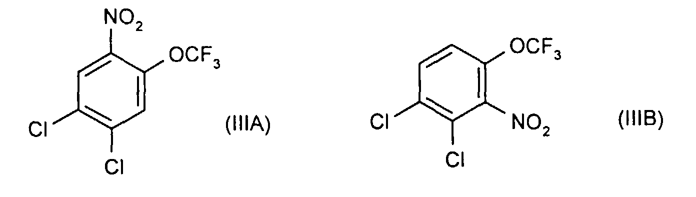 are reacted in a second step with hydrogen in the presence of a metal catalyst, in the presence of a diluent and, if appropriate, in the presence of an acid acceptor, at temperatures between 0°C and 200°C,
it being possible to isolate the intermediate dichloro-trifluoromethoxy-anilines of the general formulae (IVA) and (IVB) 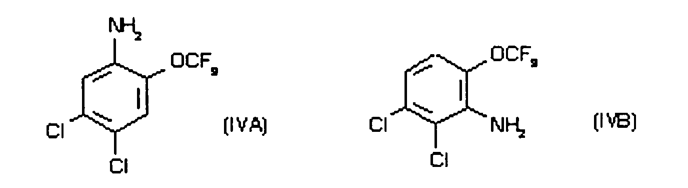 if so desired.

3. Process according to Claim 1, characterized in that the first process step is carried out at temperatures between -10°C and +60°C.

4. Process according to Claim 2, characterized in that the first process step is carried out at temperatures between 0°C and +40°C.

5. Process according to Claim 1, characterized in that the second process step is carried out at temperatures between 10°C and 150°C.

6. Process according to Claim 4, characterized in that the second process step is carried out at temperatures between 20°C and 100°C.

7. Process according to Claim 1, characterized in that the second process step is carried out at a pressure in the range of from 1 bar to 100 bar.

8. Process according to Claim 6, characterized in that the second process step is carried out at a pressure in the range of from 1 bar to 50 bar.

9. 1,2-Dichloro-5-nitro-4-trifluoromethoxy-benzene of the formula (IIIA) and 1,2-dichloro-3-nitro-4-trifluoromethoxy-benzene of the formula (IIIB)

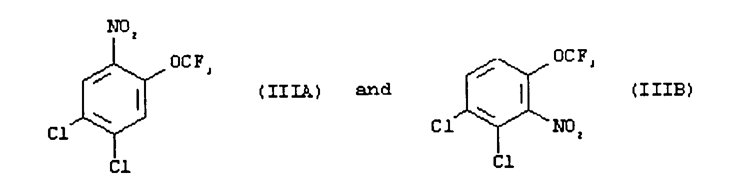

10. 4,5-Dichloro-2-trifluoromethoxy-aniline of the formula (IVA) and 5,6-dichloro-2-trifluoromethoxyaniline of the formula (IVB)

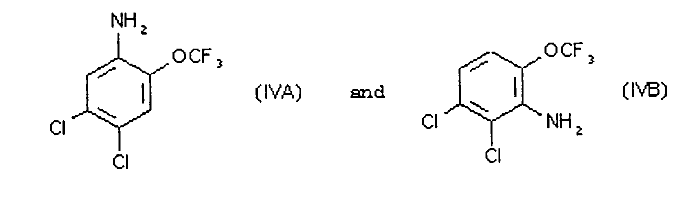

## Revendications

1. Procédé de production de 2-trifluorométhoxy-aniline de formule (I), 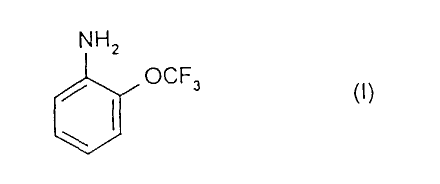 caractérisé en ce qu'on fait réagir dans une première étape le 1,2-dichloro-4-trifluorométhoxy-benzène de formule (II) 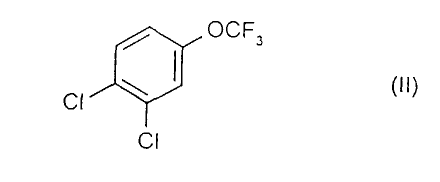 avec un agent de nitration à des températures comprises entre -20°C et +80°C
et on fait réagir les produits de nitration ainsi formés, de formules générales (IIIA) et (IIIB) 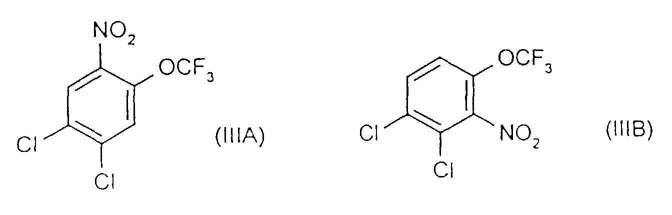 dans une seconde étape, avec l'hydrogène en présence d'un catalyseur métallique, en présence d'un diluant et à des températures comprises entre 0°C et 200°C,
les dichlorotrifluorométhoxy-anilines également formées au stade intermédiaire, de formules générales (IVA) et (IVB) 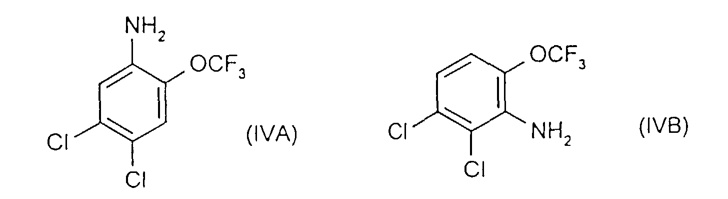 pouvant éventuellement être isolées.

2. Procédé de production de 2-trifluorométhoxy-aniline de formule (I), 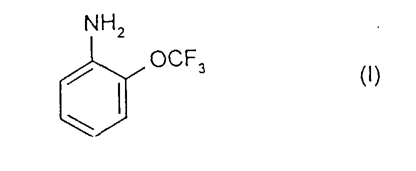 caractérisé en ce qu'on fait réagir dans une première étape le 1,2-dichloro-4-trifluorométhoxy-benzène de formule (II) 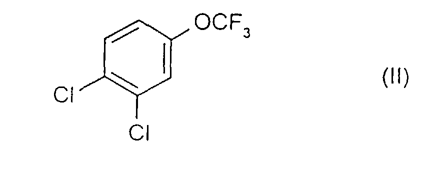 avec un agent de nitration en présence d'un acide et/ou d'un solvant organique inerte, à des températures comprises entre -20°C et +80°C
et on fait réagir dans une seconde étape les produits de nitration ainsi formés, de formules générales (IIIA) et (IIIB) 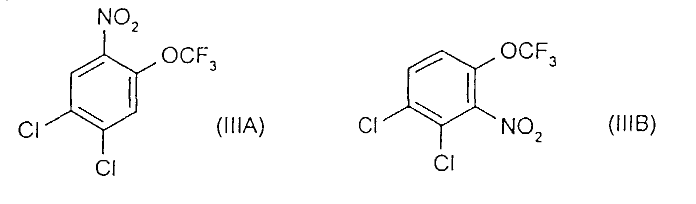 avec l'hydrogène en présence d'un catalyseur métallique, en présence d'un diluant et, le cas échéant, en présence d'un accepteur d'acide, à des températures comprises entre 0°C et 200°C,
les dichlorotrifluorométhoxy-anilines également formées au stade intermédiaire, de formules générales (IVA) et (IVB) 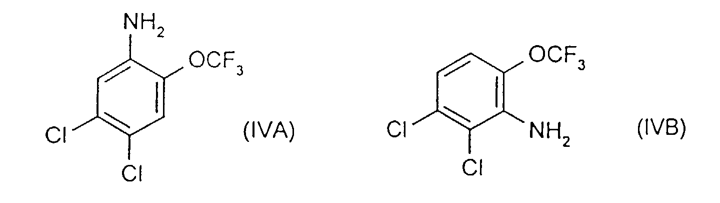 pouvant alors être isolées, le cas échéant.

3. Procédé suivant la revendication 1, caractérisé en ce que la première étape du procédé est conduite à des températures comprises entre -10°C et +60°C.

4. Procédé suivant la revendication 2, caractérisé en ce que la première étape du procédé est conduite à des températures comprises entre 0°C et +40°C.

5. Procédé suivant la revendication 1, caractérisé en ce que la seconde étape du procédé est conduite à des températures entre 10°C et 150°C.

6. Procédé suivant la revendication 4, caractérisé en ce que la seconde étape du procédé est conduite à des températures entre 20°C et 100°C.

7. Procédé suivant la revendication 1, caractérisé en ce que la seconde étape du procédé est conduite à une pression dans la plage de 1 bar à 100 bars.

8. Procédé suivant la revendication 6, caractérisé en ce que la seconde étape du procédé est conduite à une pression dans la plage de 1 bar à 50 bars.

9. Le 1,2-dichloro-5-nitro-4-trifluorométhoxy-benzène de formule (IIIBA) et le 1,2-dichloro-3-nitro-4-trifluorométhoxy-benzène de formule (IIIB)

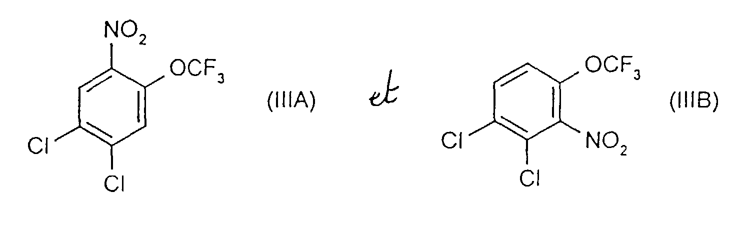

10. La 4,5-dichloro-2-trifluorométhoxy-aniline de formule (IVA) et la 5,6-dichloro-2-trifluorométhoxy-aniline de formule (IVB)
